(19) Europäisches Patentamt
European Patent Office
Office européen des brevets

(11) Veröffentlichungsnummer : **0 159 545**
**B1**

(12) # EUROPÄISCHE PATENTSCHRIFT

(45) Veröffentlichungstag der Patentschrift : **03.12.86**

(51) Int. Cl.⁴ : **C 07 C148/00**, C 07 C149/41

(21) Anmeldenummer : **85103304.3**

(22) Anmeldetag : **21.03.85**

(54) **Verfahren zur Herstellung von o,o'-Dithiobenzamiden.**

(30) Priorität : **28.03.84 DE 3411385**

(43) Veröffentlichungstag der Anmeldung : **30.10.85 Patentblatt 85/44**

(45) Bekanntmachung des Hinweises auf die Patenterteilung : **03.12.86 Patentblatt 86/49**

(84) Benannte Vertragsstaaten : **AT BE CH DE FR GB IT LI NL SE**

(56) Entgegenhaltungen :
**EP-A- 0 085 181**
**US-A- 3 786 150**

(73) Patentinhaber : **BASF Aktiengesellschaft**
**Carl-Bosch-Strasse 38**
**D-6700 Ludwigshafen (DE)**

(72) Erfinder : **Jaedicke, Hagen, Dr. Chem.**
**Anglerstrasse 38**
**D-6700 Ludwigshafen (DE)**

**EP 0 159 545 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Verfahren zur Herstellung von o,o'-Dithiobenzamiden aus Anthranilsäureamiden mit salpetriger Säure und Schwefeldioxid.

o,o'-Dithiobenzolsäureamide sind bekannt (US-A-3 736 280, US-A-3 663 616, DE-B-26 15 662 und US-A-3 786 150). Es ist bekannt, diese Verbindungen in der Weise herzustellen, daß substituierte o,o'-Dithiodiphenyldicarbonsäurechloride mit Ammoniak oder Aminen umgesetzt werden (H. Böshagen, Chem. Ber. *99*, 2566 (1966), DE-B-26 15 662). Dieses Verfahren hat den Nachteil, daß die o,o'-Dithiodiphenyldicarbonsäurechloride nur schlecht zugänglich sind.

Es wurde nun gefunden, daß man o,o'-Dithiodibenzamide der Formel

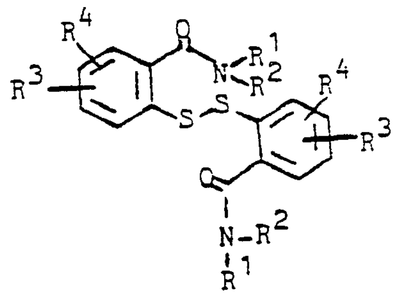

in der $R^1$ Wasserstoff, Alkyl mit 1 bis 4 Kohlenstoffatomen oder Phenyl, $R^2$ Wasserstoff, Alkyl mit 1 bis 4 Kohlenstoffatomen oder Phenyl und $R^3$ Wasserstoff, Chlor, Brom, Fluor, Nitro, Methyl oder Methoxyl, $R^4$ die gleichen Bedeutungen wie $R^3$ hat und gleich oder verschieden von $R^3$ ist bedeuten, in einfacher Weise erhält, indem man ein Anthranilsäureamid der Formel

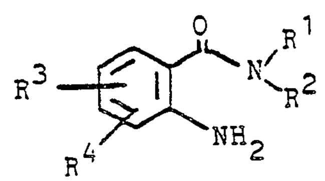

in der $R^1$, $R^2$, $R^4$ und $R^3$ die obengenannten Bedeutungen haben in Wasser nitrosiert und mit Schwefeldioxid in Gegenwart von Kupfer, einer Kupferverbindung oder einem Kupfersalz umsetzt. Auf diese Weise erhält man aus dem einfachen Ausgangsprodukt Anthranilsäureamid, das leicht aus Isatosäureanhydrid mit Ammoniak oder Aminen zugänglich ist, das gewünschte Endprodukt.

Alkyl mit 1 bis 4 Kohlenstoffatomen ist beispielsweise Methyl, Ethyl, Propyl, Isopropyl, Butyl, sek.-Butyl, tert.-Butyl.

Nitrosierung ist beispielsweise die Umsetzung mit salpetriger Säure, Salpetrigsäureester (z. B. Amylnitrit) oder die Umsetzung mit nitrosen Gasen.

Eine Kupferverbindung ist beispielsweise Kupferoxid. Ein Kupfersalz ist beispielsweise Kupfer-IIchlorid, Kupfer-II-sulfat oder Kupfer-II-nitrat.

Die Umsetzung mit salpetriger Säure erfolgt beispielsweise durch Umsetzung mit Natriumnitrit und einer Mineralsäure, z. B. Salzsäure oder Schwefelsäure. Das erfindungsgemäße Verfahren kann beispielsweise durch die folgende Skizze dargestellt werden, in der X das Anion einer Mineralsäure bedeutet.

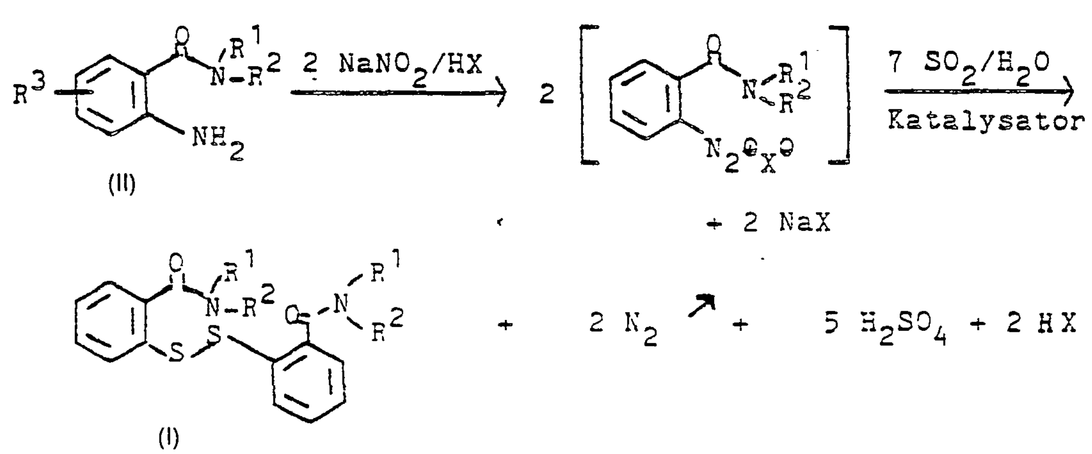


(II)

(I)

Der Katalysator kann beispielsweise ein Cu-Salz sein (Chlorid/Sulfat/Nitrat), oder metallisches Cu. Man kann das Cu-Salz oder Cu auch kombinieren mit einem Alkalijodid. Man verwendet beispielsweise 0,001 bis 0,1, vorzugsweise 0,01 Mole Cu-Salz pro Mol der Verbindung II und beispielsweise Alkalijodid in gleicher Menge, wenn man die Mischung verwendet. Geeignet für die Mischung sind auch elementares Jod oder Jodwasserstoff.

Die Umsetzung mit salpetriger Säure wird beispielsweise bei 0 °C bis 40 °C, vorzugsweise bei 5 °C bis 10 °C, vorgenommen. Die Zersetzung wird beispielsweise bei 10 bis 100 °C vorzugsweise bei 40 bis 70 °C vorgenommen. Das $SO_2$ kann z. B. zusammen mit dem Diazoniumsalz zu dem Katalysator oder zu der Mischung aus Diazoniumsalz und Katalysator geleitet werden.

Die folgenden Beispiele erläutern die Durchführung des Verfahrens.

Beispiel 1

108,8 g Anthranilsäureamid wurden in 975 g 30 %iger (Gew.-%) Salzsäure suspendiert. Man kühlte auf 5 °C und tropfte innerhalb von 30 Minuten 223,5 g einer 25 %igen $NaNO_2$-Lösung in Wasser zu. Man rührte noch 30 minuten bei 5 °C und pumpte die gekühlte Lösung dann innerhalb von 5 Stunden zu einer Lösung, die aus 340 ml $H_2O$, 1,4 g $CuCl_2 \cdot 2\ H_2O$ und 1,3 g KJ und 5 g $SO_2$ hergestellt worden war (Katalysatorlösung). Die Katalysatorlösung war auf 50 °C erwärmt worden. Gleichzeitig mit dem nitrosierten Anthranilamid leitete man einen Strom von 46 g $SO_2$/Std. in die Reaktionsmischung. Nach beendetem Zulauf des nitrosierten Anthranilamids wurde die Suspension von o,o'-Dithiodibenzamid noch eine Stunde bei 55 °C gerührt. Nach Abkühlen, Absaugen und Trocknen erhielt man 110,5 g reines o,o'-Dithiodibenzamid vom Schmelzpunkt 252 bis 253 °C.

Beispiel 2

o,o'-Diphenyldisulfiddicarbonsäure-diisopropylamid

178 g Anthranilsäureisopropylamid wurden in 1 100 g Salzsäure (30 %ig) suspendiert. Bei 10 bis 15 °C tropfte man 173 g einer 40 %igen wäßrigen Lösung von $NaNO_2$ zu. Die Temperatur von 15 °C wurde nicht überschritten. Man erhielt eine Suspension, die innerhalb von zwei Stunden gleichmäßig in ein gerührtes Zersetzungsgefäß gepumpt wurde. Dort war eine Lösung von 3,5 g $CuCl_2 \cdot 2\ H_2O$ und 1,5 g KJ in 100 ml Wasser vorgelegt worden. Während der ersten Stunde Pumpzeit leitete man 192 g $SO_2$, in der zweiten Stunde 96 g $SO_2$ in das Zersetzungsgefäß. Die Temperatur betrug in dieser Zeit gleichmäßig 55 °C. Man rührte nach beendetem Zulauf noch eine Stunde bei 95 °C, kühlte dann ab und erhielt 163 g o,o'-Dithiodibenzoesäure-diisopropylamid vom Fp 244 bis 245 °C (Lit. Fp. 245 °C ; F. Gialdi, R. Pouci und A. Baruffini, Farmaco Ed. Sci. *14*, 648 (1959)).

Beispiel 3

108,8 g Anthranilsäureamid wurden in 1 100 g einer 70 Gew.-%igen $H_2SO_4$ gelöst. Bei 25 bis 30 °C tropfte man 223,5 g 25 %ige wäßrige $NaNO_2$-Lösung zu. Dann rührte man eine Stunde bei 25 °C. Die Lösung floß in vier Stunden in ein Gefäß, das auf 60 °C temperiert war und eine Lösung von 1,1 g KJ und 2,1 g $CuSO_4 \cdot 5\ H_2O$ in 150 ml $H_2O$ enthielt. Gleichzeitig mit dem Zufluß der Lösung des Diazoniumsalzes gaste man 230 g $SO_2$ in das Gefäß. Die Suspension wurde nach beendetem Zulauf noch eine Stunde bei 60 °C gerührt und dann abgesaugt. Man erhielt 113,4 g reines o,o'-Dithiodibenzamid (Fp 252 bis 253 °C).

**Patentansprüche**

1. Verfahren zur Herstellung von o,o'-Dithiobenzamiden der Formel

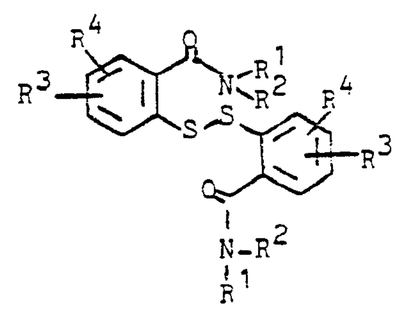

in der

$R^1$ Wasserstoff, Alkyl mit 1 bis 4 C-Atomen oder Phenyl,
$R^2$ Wasserstoff, Alkyl mit 1 bis 4 C-Atomen oder Phenyl,
$R^3$ Wasserstoff, Chlor, Brom, Fluor, Nitro, Methyl oder Methoxyl und
$R^4$ die gleichen Bedeutungen wie $R^3$ hat und gleich oder verschieden von $R^3$ ist

bedeutet, dadurch gekennzeichnet, daß man ein Antranilsäureamid der Formel

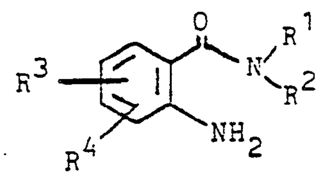

in der $R^1$, $R^2$ und $R^3$ die oben genannten Bedeutungen haben in Wasser nitrosiert und mit Schwefeldioxid in Gegenwart von Kupfer, einer Kupferverbindung oder einem Kupfersalz umsetzt.

2. Verfahren nach Anspruch 1, dadurch gekennzeichnet, daß man die Umsetzung mit Schwefeldioxid in Gegenwart eines Gemisches aus einem Kupfersalz und Jod, Jodwasserstoff oder einem Alkalijodid durchführt.

3. Verfahren nach Anspruch 1, dadurch gekennzeichnet, daß man die Umsetzung mit Schwefeldioxid bei 10 bis 100 °C durchführt.

**Claims**

1. A process for the preparation of an o,o'-dithiodibenzamide of the formula

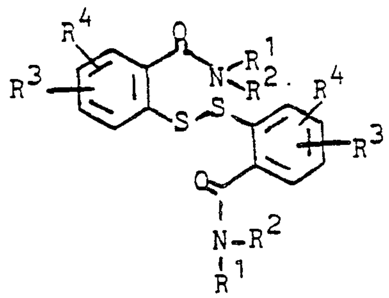

where
$R^1$ is hydrogen, alkyl of 1 to 4 carbon atoms or phenyl,
$R^2$ is hydrogen, alkyl of 1 to 4 carbon atoms or phenyl,
$R^3$ is hydrogen, chlorine, bromine, fluorine, nitro, methyl or methoxy, and
$R^4$ has the same meanings as $R^3$ and is identical to or different from $R^3$,

wherein an anthranilamide of the formula

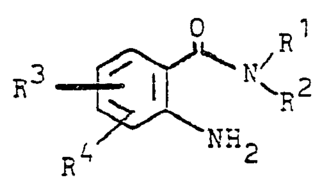

where $R^1$, $R^2$ and $R^3$ have the above meanings, is nitrosated in water, and the product is reacted with sulfur dioxide in the presence of copper, a copper compound or a copper salt.

2. A process as claimed in claim 1, wherein the reaction with sulfur dioxide is carried out in the presence of a mixture of a copper salt and iodine, hydrogen iodide or an alkali metal iodide.

3. A process as claimed in claim 1, wherein the reaction with sulfur dioxide is carried out at from 10 to 100 °C.

**Revendications**

1. Procédé pour la préparation de o,o'-dithiobenzamides de formule

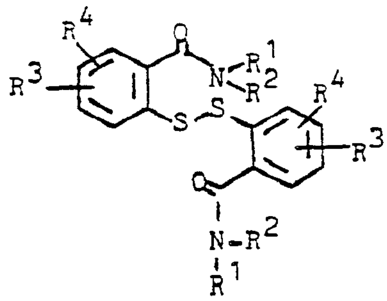

dans laquelle

$R^1$ représente hydrogène, alkyle de 1 à 4 atomes C ou phényle,

$R^2$ hydrogène, alkyle de 1 à 4 atomes C ou phényle,

$R^3$ hydrogène, chlore, brome, fluor, nitro, méthyle ou méthoxyle et

$R^4$ a les mêmes significations que $R^3$ et est identique ou différent de $R^3$

caractérisé par le fait que l'on nitre, dans l'eau, un amide d'acide antranilique de formule

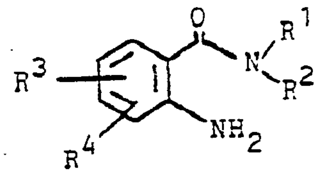

dans laquelle $R^1$, $R^2$ et $R^3$ ont les significations sus-indiquées, et on le fait réagir avec de l'anhydride sulfureux, en présence de cuivre, d'un composé du cuivre ou d'un sel de cuivre.

2. Procédé selon la revendication 1, caractérisé par le fait que l'on effectue la réaction avec l'anhydride sulfureux en présence d'un mélange d'un sel de cuivre et d'iode, d'acide iodhydrique ou d'un iodure alcalin.

3. Procédé selon la revendication 1, caractérisé par le fait que l'on effectue la réaction avec l'anhydride sulfureux à une température de 10 à 100 °C.